# EUROPEAN PATENT APPLICATION

(11) **EP 2 423 881 A2**
(43) Date of publication of application: **29.02.2012**
(21) Application number: 11178606.7
(22) Date of filing: 24.08.2011
(51) Int. Cl.: G06T 11/60, G06T 3/40

(54) **Image processing apparatus and method**

(30) Priority: 31.08.2010 JP 2010193845
(71) Applicant: CANON KABUSHIKI KAISHA, Ohta-ku Tokyo 146-8501 (JP)
(72) Inventor: Takekoshi, Koji, Ohta-ku, Tokyo (JP)
(74) Representative: Northway, Daniel Robert

(57) **Abstract**

An image processing apparatus that performs a position adjustment on an image displayed on a display screen (106) includes an obtaining unit (101, 102, 103, 104) configured to obtain a plurality of images for which mutual positional relationships are set, a display control unit (105) configured to display at least one image among the plurality of images on the display screen, a first position adjustment unit (105) configured to adjust a position of the image displayed on the display screen, and a second position adjustment unit (105) configured to adjust a position of the image that is not displayed on the display screen among the plurality of images in accordance with the adjustment of the position by the first position adjustment unit.

## Description

### BACKGROUND OF THE INVENTION

### Field of the Invention

The present invention relates to an image processing apparatus and method.

### Description of the Related Art

In recent years, for an X-ray image pickup apparatus for medical purposes, digital X-ray image pickup apparatuses using various methods are being widely spread along with developments in digital technologies. For example, a digital X-ray image pickup apparatus is put into practical use for carrying out a direct digitalization of an X-ray image without intermediation of an optical system or the like by using an X-ray planar detector in which a fluorescent member is in close contact with a large-area amorphous silicon (a-Si) sensor. The X-ray planar detector is so-called flat panel detector (FPD).

The digital X-ray image pickup apparatus is widely used for obtaining a diagnostic image, but it is also necessary to pick up an image for an area of the subject (e.g. the entire backbone or the entire lower limb) of which the size is beyond a size of a detection surface of the digital X-ray image pickup apparatus.

To obtain a picked-up image of the entire backbone or the entire lower limb of the subject, first, picking up of an image is carried out plural times while the image pickup target is divided into a plurality of areas. Then, by joining a plurality of obtained adjacent picked-up images with one another, the picked-up image of the entire backbone or the entire lower limb is obtained.

Japanese Patent Laid-Open No. 2005-261666 discloses a technique for creating a diagnostic image by combining a plurality of picked-up images with one another.

However, in a case where the image pickup is carried out plural times, a body motion of the subject occurs during the image pickup, and a position shift may occur between the plurality of picked-up images in some cases. A problem of the position shift between the picked-up images also develops at the time of creating not only the digital X-ray image pickup apparatus but also a panoramic combined picture or the like. As an example of technology for adjusting this position shift on the basis of a user instruction, Japanese Patent No. 3542898 discloses a technology for carrying out an adjustment processing such as a movement, a rotation, an expansion, a reduction, or a deformation with respect to a picked-up image group.

According to the technology disclosed in Japanese Patent No. 3542898, for example, when an adjustment for the position shift of four images is carried out, by interlocking two images, time and effort for the position adjustment are reduced.

However, according to the technology disclosed in Japanese Patent No. 3542898, it is necessary for a user to explicitly specify a plurality of images to be interlocked with one another. Also, to appropriately specify the images to be interlocked with one another, all the images that become movement targets need to be displayed on a display screen. For that reason, in a case where all the images that become the movement targets are not displayed because of an expanded display, a reduced display needs to be carried out for the interlock specification, which increases the time and effort of the user.

### SUMMARY OF THE INVENTION

It is desirable to reduce time and effort with regard to an interlock specification for a plurality of images in an image processing apparatus for carrying out a position adjustment on the images displayed on a display screen.

The present invention in its first aspect provides an image processing apparatus as specified in claims 1 to 8.

The present invention in its second aspect provides an image processing method as specified in claims 9 and 10.

The present invention in its third aspect provides a computer program as specified in claims 11 and 12, and a storage medium containing the computer program as specified in claim 13.

Further features of the present invention will become apparent from the following description of exemplary embodiments with reference to the attached drawings.

### BRIEF DESCRIPTION OF THE DRAWINGS

The accompanying drawings, which are incorporated in and constitute a part of the specification, illustrate embodiments of the invention and, together with the description, serve to explain the principles of the invention, though the invention is not limited to what is disclosed therein.

FIG. 1 illustrates a configuration of an X-ray image pickup apparatus according to an embodiment.

FIG. 2 is a flow chart for a long image pickup processing according to the present embodiment.

FIGS. 3A and 3B illustrate an image processing for an image combining.

FIG. 4 illustrates an example of a position adjustment screen according to an embodiment.

FIG. 5 is a flow chart for a detail of a position adjustment processing according to an embodiment.

FIG. 6 is an explanatory diagram for describing a processing in a case where an expansion operation is carried out.

FIGS. 7A to 7E illustrate patterns of a positional relation between the position adjustment screen and a plurality of picked-up images.

### DESCRIPTION OF THE EMBODIMENTS

Preferred embodiments of the present invention will be described in detail in accordance with the accompanying drawings.

### First Embodiment

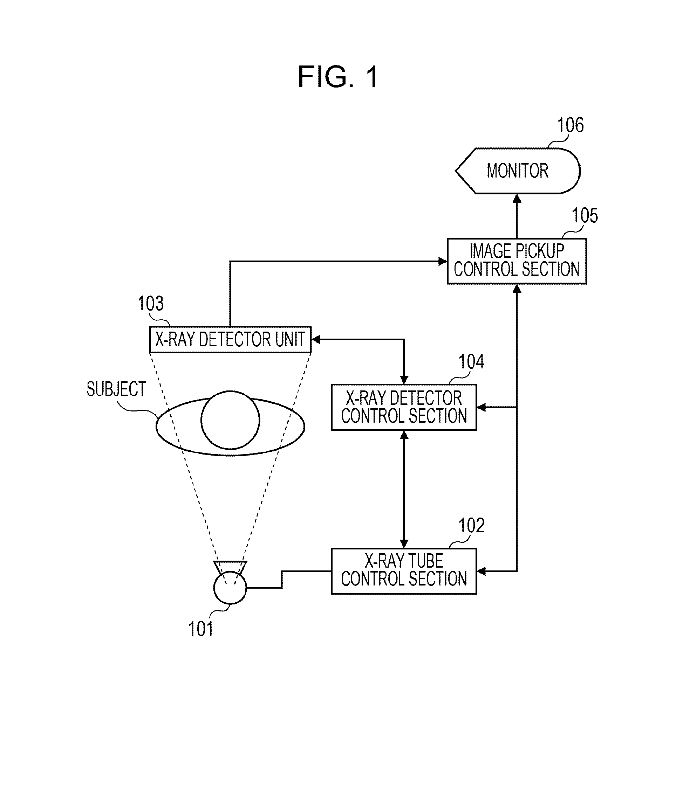

FIG. 1 illustrates a configuration of an X-ray image pickup apparatus according to a first embodiment. The X-ray image pickup apparatus has the following configuration.

An X-ray tube 101 irradiates a subject with X-rays.

An X-ray tube control section 102 controls an irradiation amount, an irradiation timing, and the like of the X-ray of the X-ray tube 101.

An X-ray detector unit 103 is an X-ray detection unit that detects the X-ray transmitting through the subject.

An X-ray detector control section 104 controls an operation of the X-ray detector unit 103.

An image pickup control section 105 controls the X-ray tube control section 102 and the X-ray detector control section 104. The image pickup control section 105 also performs image processing on an X-ray picked-up image obtained from the X-ray detector unit 103, a display control on the X-ray picked-up image onto a monitor 106, a saving processing of the X-ray picked-up image, and the like. The image pickup control section 105 can have a configuration similar to a general personal computer having a CPU (central processing unit), a RAM (random access memory), and the like, and be run by a computer program to cause the control of the X-ray tube control section and of the X-ray detector control section.

Next, an X-ray long image pickup processing using the X-ray image pickup apparatus of FIG. 1 will be described.

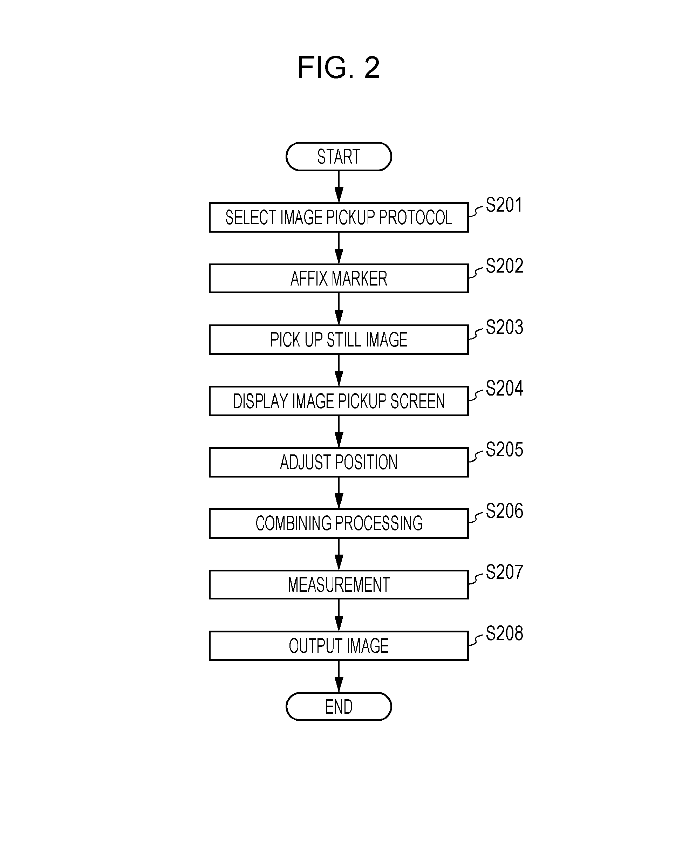

FIG. 2 is a flow chart for the X-ray long image pickup processing according to the present embodiment. Respective processings in the flow chart of FIG. 2 are carried out by the image pickup control section 105 or an operator of the X-ray image pickup apparatus.

(Step S201) In step S201, through an instruction of the operator with respect to the image pickup control section 105, a selection of an image pickup protocol is carried out.

The image pickup protocol according to the present embodiment refers to various image pickup conditions previously set for the X-ray image pickup. To be more specific, the image pickup protocol includes control information for controlling the X-ray detector, control information for controlling the X-ray tube control section, and the like. Furthermore, the image pickup protocol includes information on an image processing condition with respect the image obtained as the result of the image pickup by the image pickup control section and a display condition for displaying the image, and the like.

Also, as a case is conceivable in which a plurality of X-ray detectors exists, the image pickup protocol further includes information for identifying the X-ray detector, orientations of vertical and horizontal directions of the X-ray detector, and the like. In particular, in the long image pickup, it is possible to previously set information for identifying the X-ray detector that performs the image pickup and vertical and horizontal orientations of the relevant X-ray detector as well. Furthermore, it is possible to set information on image pickup parts such as the entire backbone and the lower limb entire length, and depending on an image pickup part, the number of images to be picked up. Herein, the maximum number of images to be picked up is set as four, but no problem occurs even when the image pickup for four or fewer images or five or more pages can be carried out.

(Step S202) In step S202, the operator affixes a marker to the subject being X-rayed, or to the X-ray detector unit for the long image pickup. A plurality of picked-up images are obtained for the long image pickup, and to grasp a relative relationship between the respective picked-up images, the marker affixing is carried out on the subject or the X-ray detector unit 103. In this way, the position of the marker in the plurality of images indicates where in the overall subject image (or "long image") each picked-up image is positioned. The marker is preferably arranged in an area functioning as a seam between the respective picked-up images (i.e. in an overlapping area between the picked-up images) and becomes a reference for combining the respective picked-up images.

For the marker, a lead sphere having a diameter of several millimeters is used in many cases. In a case where the long image pickup using the marker is carried out, a radiation image pickup support for efficiently and accurately picking up an image of the marker at the time of the image pickup may also be used. As a substitute for the marker, a magnet for a magnetic therapy, high-purity germanium, or the like may be used. Also, a shotgun shell or the like may be used as a substitute.

It is noted that step S201 and step S202 described above are pre-processings for the image pickup processing, and the processing order may be reversed.

(Step S203) In step S203, the subject is irradiated with the X-ray to carry out an X-ray image pickup process. While following the image pickup protocol set in step S201, the X-ray image pickup process is carried out as the image pickup control section 105 controls the X-ray image pickup apparatus. After the image pickup protocol selection, in accordance with the size of the image pickup area of the subject, a control on the number of images to be picked up may also be carried out. In the long image pickup, as the image pickup is carried out for the plurality of areas of the subject, a position of the X-ray detector unit 103 and an X-ray irradiation direction from the X-ray tube 101 are changed during the image pickup. For example, in a case where the image pickup is carried out while the subject is in a standing position, the X-ray detector unit 103 is moved in the vertical direction by a driving unit which is not illustrated in the drawing, and the X-ray irradiation direction of the X-ray tube 101 is changed while the movement is followed. On the other hand, a relative positional relation between a focus of the X-ray tube 101 and the subject is not changed.

(Step S204) In step S204, the image pickup control section 105 displays a plurality of picked-up images obtained in step S203 in a display screen such as a monitor. The plurality of picked-up images displayed herein are subjected to a registration while the above-described marker is used as a reference. The image pickup control section 105 functions as an image processing unit that performs an image processing for extracting the marker from the plurality of picked-up images and a display control unit that displays the picked-up images in the display screen. According to the present embodiment, with regard to the respective picked-up images, as the marker appears in the images, it is possible to calculate the relative positional relationship of the picked-up images by extracting the marker through image processing. The combining of the plurality of picked-up images may be carried out by extracting a position of the marker from the picked-up images by the image pickup control section 105 to perform the position adjustment on the respective picked-up images or through a manual operation based on the instruction of the operator.

In a case where the extraction of the marker fails, the picked-up images are displayed while being overlapped at a previously set value. For example, an overlapping amount can be set as 48 mm, 50 mm, or the like.

Also, without using the marker, the image pickup control section 105 may calculate the overlapping amount of the images from a relative positional relation between an X-ray detector-holding section that holds the X-ray detector unit 103 and an X-ray tube-holding section that holds the X-ray tube 101 and the image pickup control section 105 may perform the position adjustment on the respective picked-up images by using the overlapping amount.

(Step S205) In step S205, the position adjustment on the plurality of picked-up images displayed in step S204 is carried out on the basis of an instruction of the operator. The operator sends the instruction to the image pickup control section 105 from an input apparatus such as a keyboard or a mouse, and the image pickup control section 105 receiving the instruction functions as a first position adjustment unit and performs the position adjustment on the picked-up images. This is because the mutual positional relation between the plurality of picked-up images is set in step S204, but a small adjustment with respect to the position shift might need to be performed because of a body motion of the subject or the like. The operator performs a mouse operation by selecting an image while the positional relation between the picked-up images displayed in the display screen is checked, or the operator inputs a numerical value from the key board so that the position of the image is moved upward and downward and to the left and the right.

As the X-ray picked-up images are used for medical diagnostics, the images may be subjected to an expanded display for carrying out a close-up observation in some cases. However, when the expanded display is carried out, some picked-up images among the plurality of picked-up images that are the combining targets may be located outside the display screen in some cases. In a case where the position adjustment on the picked-up images is carried out in the above-described situation, as the position on the picked-up image outside the display screen is unknown, a reduced display is carried out after the position adjustment, and the positional relation between the entire picked-up images needs to be checked again. Therefore, according to the present embodiment, in a case where the image pickup control section 105 functions as a second position adjustment unit and the position adjustment on the picked-up images is carried out, the position on the picked-up image that is not displayed on the display screen is also interlocked and adjusted. With this function, it is possible to alleviate the time and effort of the operator who normally performs the position adjustment. Also, in a case where the position adjustment amount by the mouse operation or the like is larger than a predetermined amount, irrespective of whether the image is in the display screen or not, the positions of the plurality of picked-up images may be adjusted. A detailed processing of the second position adjustment unit will be described below.

(Step S206) In step S206, the image pickup control section 105 functions as a combining processing unit and performs a combining processing on the plurality of picked-up images. In a case where the operator presses a position decision button, the image pickup control section 105 performs the combining processing. The positional relationship between the plurality of picked-up images that are the combining targets is adjusted through the processings in step S204 and step S205. Therefore, the image pickup control section 105 joins seams between the plurality of picked-up images of which positions are adjusted (combining processing) to generates a single combined image. The generated combined image is displayed on the monitor 106. It is noted that in a case where the combining processing in the present step is carried out, an interlocking processing in step S205 may be restricted.

(Step S207) In step S207, the image pickup control section 105 performs a measurement processing. Various measurements such as a Cobb angle measurement and a high difference measurement can be performed on the combined image displayed on the monitor 106. Herein, a display based on a value obtained by correcting an X-ray expansion effect is preferably carried out. For the correction of the X-ray expansion effect, the value may be obtained through the calculation of the respective relative positional relationships between the sensor, the subject, and the X-ray tube, or a method of specifying a correction value of the expansion effect through the instruction of the operator may also be employed.

(Step S208) In step S208, the image pickup control section 105 outputs the combined image to an external apparatus. For an output of the image, in general, the image is output through a communication protocol defined by a standard called DICOM (Digital Imaging and Communication in Medicine). For the DICOM output, a printing output or the like to an image server called PACS (Picture Archiving and Communication System) or a film imager for medical purposes can be carried out, and it is possible to output both the picked-up images before the combining and the combined image to the PACS. Also, depending on a setting, a case exists in which only the picked-up images are output or a case exists in which only the combined image is output. Also, in the case of the printing output, only the combined image is output. The above-described respective steps can also be realized while software (computer program) obtained via a network or various storage media is executed by a processing apparatus such as a personal computer (or CPU, processor, etc.).

### (Detail of position adjustment processing in Step S205)

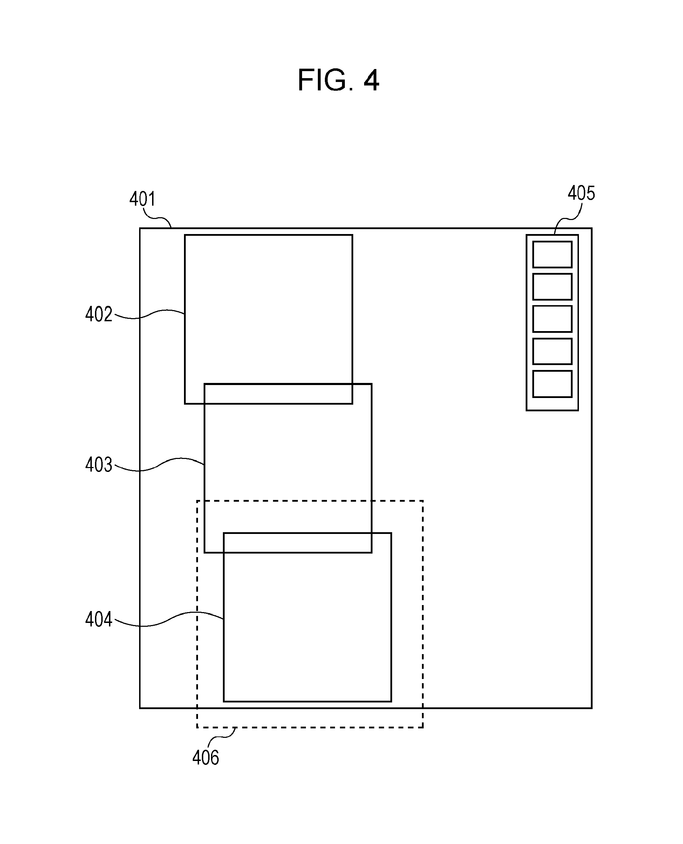

FIG. 4 illustrates an example of a position adjustment screen according to the present embodiment. Picked up images 402, 403 and 404 displayed on a position adjustment screen 401 are combined with one another to generate a long picked-up image (i.e. a combined image). Herein, three images are exemplified for the description, but the number of images is not limited to three. A tool group 405 is used for carrying out an image operation. For example, the tool group includes an image fit button, a maximum expansion button, an expansion button, a reduction button, a free rotation button, a right 90-degree rotation button, a left 90-degree rotation button, a cutout button, an Undo button, an up, down, left, and right movement button, an edge emphasis button, and the like.

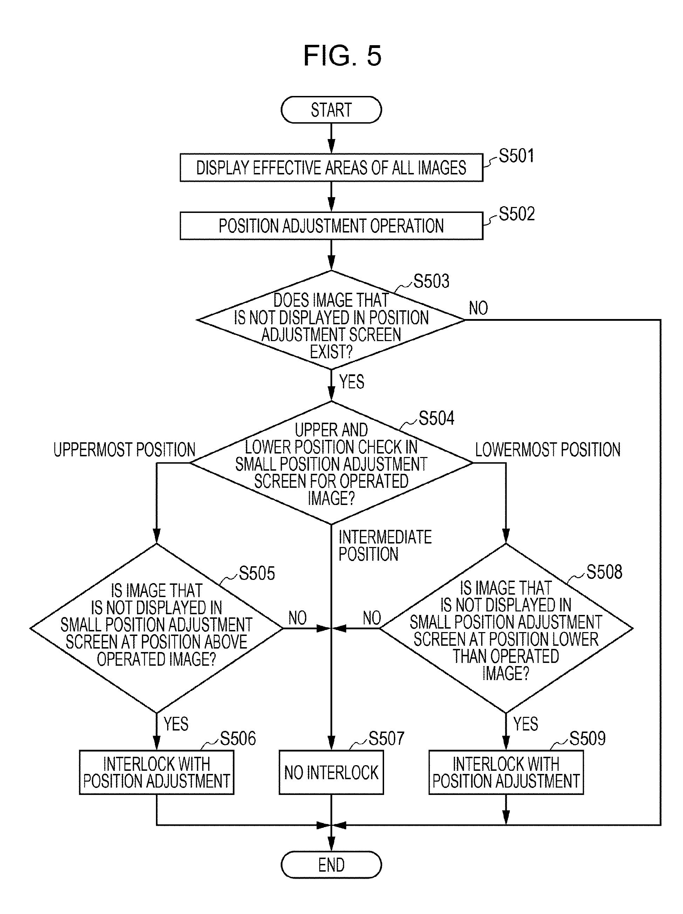

A detail of a position adjustment processing will be described. FIG. 5 is a flow chart for the detail of the position adjustment processing according to the present embodiment. It is noted that processings in the following respective steps are carried out by the image pickup control section 105 or the operator of the X-ray image pickup apparatus.

(Step S501) In step S501, the image pickup control section 105 displays an effective area of all the picked-up images. The effective area refers to an area significant in terms of diagnostics where the X-ray is irradiated over the entire image surface. For the diagnostics or the like, the effective area on the picked-up images is generally used. The respective picked-up images displayed on the position adjustment screen 401 on the monitor 106 become respective effective areas of the plurality of picked-up images.

For example, the image 404 is an effective area of an image 406 (the image 402 and the image 403 are respectively the effective image areas. Actual image areas for these effective image areas are not illustrated in the drawing). That is, the image 406 is an actual image size, and an X-ray irradiation area of this actual image is the image 404. The X-ray irradiation area can be decided by using a technology in related art.

(Step S502) In step S502, after the instruction of the operator is received, the image pickup control section 105 functions as a first position adjustment unit and performs a position adjustment operation. The position adjustment corresponds to the up, down, left, and right movement and the rotation operation by using the image operation tool group 405 or the like. Although other image operations are also carried out, the target in step S502 is one accompanying the position change of the image or geometric conversion. After the processing in the present step is carried out, the processing advances to step S503.

(Step S503) In step S503, the image pickup control section 105 functions as a determination unit and performs a determination as to whether or not a further image exists that is not displayed in the position adjustment screen 401. In a case where the position adjustment is carried out, the expansion operation is carried out in many cases prior to the position adjustment.

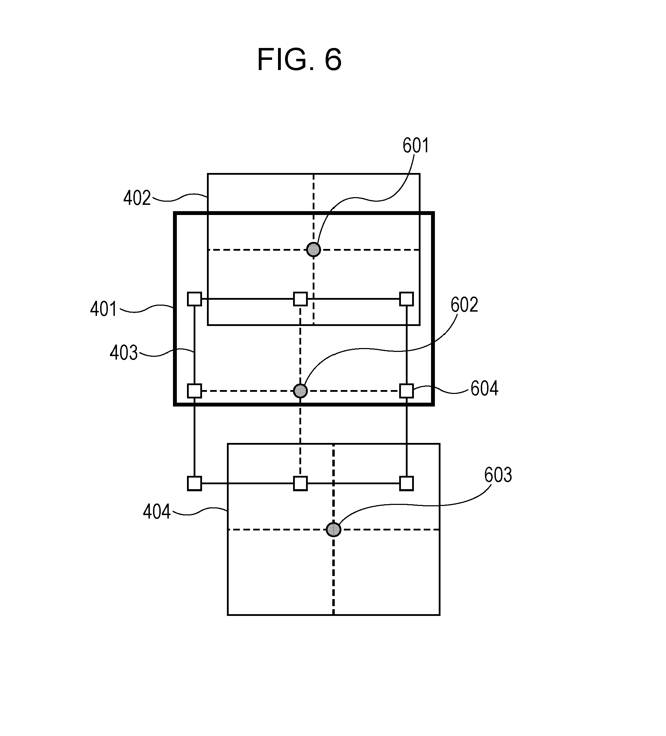

FIG. 6 is an explanatory diagram for describing a processing in a case where the above-described expansion operation is carried out.

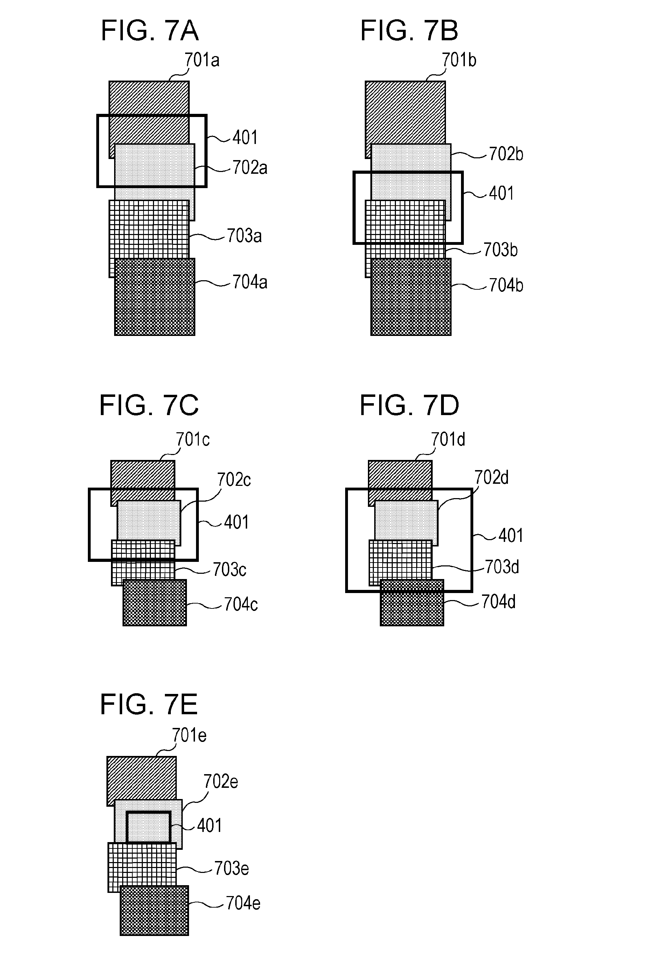

In a case where the image expansion operation is carried out, the image is expanded while the relative positional relation between the images is maintained. For that reason, when the expansion is carried out to see the detail of the overlapping area between the two images, the other overlapping area protrudes from the screen. As a result of the expansion for checking the overlapping area of the image 402 and the image 403, the image 404 is pushed out of the area of the position adjustment screen 401 and no image is displayed in the screen of the image 404. Therefore, in a case where it is determined that an image exists that is not displayed on the position adjustment screen, the existing image being among the images 402 to 404, the images 402 and 403 are displayed, and the image 404 becomes the image that is known but not displayed. In a case where the image exists that is not displayed on the position adjustment screen, the processing advances to step S504. It is noted that in a case where the image that is not displayed on the position adjustment screen exists, the processing is ended. Herein, the determination processing in the present step S503 will be described. FIGS. 7A to 7E illustrate patterns of the positional relation between the position adjustment screen 401 and the plurality of picked-up images.

FIGS. 7A, 7B, and 7E illustrate a state in which at least two picked-up images are located outside the position adjustment screen 401. FIG. 7C illustrates a state in which at least one picked-up image is located outside the position adjustment screen 401. In such cases, the processing advances to step S504.

On the other hand, in FIG. 7D, at least a part of all the picked-up images is displayed on the position adjustment screen 401. Therefore, in the case of the positional relation illustrated in FIG. 7D, the processing is ended.

(Step S504) In step S504, the image pickup control section 105 functions as the determination unit and determines the upper and lower positions in the position adjustment screen 401 on the picked-up images that are the operation targets for the position adjustment. This processing is for determining a relative position of the currently-operated image among the picked-up images displayed in the position adjustment screen 401. This determination will be described by using FIG. 6.

In FIG. 6, when it is supposed that the image 403 is the picked-up image of the operation target, a handle 604 for the operation is displayed in the image. Herein, the handle 604 for the operation is displayed for representing that the operation is being carried out, but other than the use of the handle for the operation, various methods are conceivable: for example, an image frame is attached, and a color of the image is changed, etc. To determine at which position the currently operated image 403 is located in the position adjustment screen, a center of each of the images is obtained. A center 601 corresponds to the center of the effective area of the image 402. A center 602 corresponds to the center of the image 403. A center 603 corresponds to the center of the image 404, but the image 404 becomes the image that is not displayed in the position adjustment screen in step S503, and an extraction of the center may be omitted.

From the above, the centers of the images displayed in the position adjustment screen correspond to the centers 601 and 602. In this example, the two images are displayed in the position adjustment screen, but a case is also conceivable in which three or more images may be displayed. As the center 602 corresponds to the center of the currently operated image and with regard to the centers 601 and 602, the center 602 is at a lowermost position as viewed in the vertical axis direction, the picked-up image of the operation target is determined to be at the lowermost position.

As a result of this determination, in a case where it is determined that the operation image is at an uppermost position among the displayed images, the processing advances to step S505. In a case where the operation image is not at the uppermost position or the lowermost position, it is determined that the operation image is at an intermediate position, and the processing advances to step S507. In a case where it is determined that the operation image is at the lowermost position among the displayed images, the processing advances to step S508.

(Step S505) In step S505, the image pickup control section 105 functions as the determination unit and determines the picked-up images operated to be interlocked.

This is a process performed in a case where it is determined in step S504 that the currently operated image is at the uppermost position. To be more specific, whether the image that is not displayed in the position adjustment screen also checked in step S503 is interlocked is decided depending on whether the relevant image is on an upper side or a lower side with respect to the operation image. A further description will be given by using FIGS. 7A to 7E. In the case of FIG. 7A, when it is supposed that an image 701a is the currently operated image, images that are not displayed in the position adjustment screen 401 are images 703a and 704a. Image 702a is displayed in the position adjustment screen 401. Therefore, a check is conducted on whether each of the images is at an upper position with respect to the currently operated image 701a. In this example, the image 703a and the image 704a are both at lower positions with respect to the operation screen and are not set as the interlocking targets. In this case where the images are not set as the interlocking targets, the processing advances to step S507.

In the case of FIG. 7B, if it is assumed that an image 702b is being operated, images that are not displayed in the position adjustment screen 401 are an image 701b and an image 704b. Image 703b is displayed in the position adjustment screen 401. Therefore, the image 701b is at an upper position with respect to the picked-up image of the operation target and is set as the interlocking target. On the other hand, the image 704b is not set as the interlocking target.

In the case of FIG. 7E, it is assumed that an image 702e is being operated. In the check on the upper and lower positions in the position adjustment screen in step S504, it is possible to determine that the position is the uppermost position and also the lower position because it is the only image displayed in the position adjustment screen 401. Images that are not displayed in the position adjustment screen 401 are an image 701e, an image 703e, and an image 704e. The image located at an upper position with respect to the picked-up image of the operation target is the image 701e, and the image 701e is set as the interlocking target.

(Step S506) In step S506, the image pickup control section 105 functions as the determination unit and performs a processing interlocked with the position adjustment. While being interlocked with the position adjustment operation with respect to the picked-up image that becomes the operation target, the position on another picked-up image is moved together with the operation target image. For the position adjustment operation, an up, down, left, and right movement of the image and a free rotation are conceivable. In this case, the operation is carried out as if the operation screen and the interlocking target image are subjected to a grouping into a single image. It is noted that for the 90-degree rotation, in the case of the interlocking target, the operation may be carried out as if the interlocking target is subjected to the grouping into the single image, or the operation may be carried out as if the interlocking target is individually rotated about the image even in the case of the interlocking target.

The interlocking operation is not cancelled until the series of the operations are ended. For example, in FIG. 7A, if it is assumed that the operation target image is an image 702a, the image 703a is the interlocking operation target. Herein, when the operation target image 702a is moved in the upper direction through a drag operation of the mouse or the like, the image 703a is interlocked and operated to be moved together in the upward direction. Then, with this movement, the image 703a may be displayed in the position adjustment screen 401 in some cases. If the image 703a is displayed in the position adjustment screen 401, the image 703a becomes the image displayed in the position adjustment screen at that instance, but in a case where the series of the operations are continued like the drag operation or the like, the image 703a may not be removed from the interlocking target and kept as the interlocking target as it is until the series of the operations are ended.

It is noted that when the position adjustment operation is carried out through the drag operation or the like, a case exists in which panning of the image or the entire image is demanded instead of a small adjustment of the position. In this case, the movement amount of the mouse drag becomes large. Therefore, in a case where the movement amount of the mouse drag operation for the single operation is larger than or equal to 1/2 of the monitor size, the panning operation is determined instead of the position adjustment on the image, and the panning operation may be carried out while interlocking all the images. It is noted that the numeric value "1/2 of the monitor size" may be 2/3, and a value that can be set through the instruction of the operator may also be used.

(Step S507) In step S507, the above-described interlocking operation is not carried out. To elaborate, the picked-up image of the operation target is individually operated, and the operation is carried out without saving the positional relation with another image. The operation in step S507 is for the case in which it is determined in step S505 that the image is located on the lower side with respect to the currently operated image among the images that are not displayed in the position adjustment screen. Regarding this case, a description will be given on the basis of FIGS. 7A to 7E. In FIG. 7A, in a case where the currently operated image is the image 701a, the image 701a is at the uppermost position in the position adjustment screen, but as the image 703a and the image 704a that are not displayed exist on the lower side with respect to the currently operated image, the interlocking operation is not carried out. In another example, in FIG. 7B, when it is assumed that the image 702b is the picked-up image being operated, the image 702b is located at the uppermost position in the position adjustment screen. On the other hand, the image 701b and the image 704b are the images that are not displayed in the position adjustment screen, but as the image 701b is located above the operation screen, the image 701b is the interlocking target. As the image 704b is located on the lower side with respect to the operation screen, the interlocking operation is not carried out with that image.

Also, in step S504, a case exists in which the currently-operated image is not at the uppermost position or the lowermost position in the position adjustment screen, and in this case, the image subjected to the interlocking operation does not exist. A description will be given on the basis of FIGS. 7A to 7E. In FIG. 7C, when it is assumed that the operation target image is the image 702c, an image 701c exists that is located above the image 702c in the position adjustment screen, and an image 704c exists that is located on the lower side with respect to the image 702c. Image 703c is also within the position adjustment screen 401. Therefore, as the image 702c is not located at the uppermost position or the lowermost position, even when the operation is carried out, the image 704c that is not displayed in the position adjustment screen is not subjected to the interlocking operation.

Furthermore, for a case where the interlocking operation is not carried out, in step S508 which will be described below, when the currently operated image is located at the lowermost position among the images displayed in the position adjustment screen, the image located above the operation screen among the images that are not displayed in the position adjustment screen is not subjected to the interlocking operation. A description will be given on the basis of FIGS. 7A to 7E. In FIG. 7B, when it is assumed that an image 703b is the operation target image, the image 701b and the image 704b are the images that are not displayed in the position adjustment screen, but as the image 701b is located above the image 703b, the image 701b does not become the interlocking target.

(Step S508) In step S508, the image pickup control section 105 functions as the determination unit and determines the picked-up images subjected to the interlocking operation.

This is the processing in a case where it is determined in step S504 that the currently operated image is located at the lowermost position. To be more specific, whether or not the interlocking operation is carried out is decided depending on whether the image checked in step S503 which is not displayed in the position adjustment screen is located on the upper side or the lower side with respect to the operation screen. A description will be given on the basis of FIGS. 7A to 7E. In FIG. 7A, when it is assumed that the currently operated image is the image 702a, the currently operated image is located at the lowermost position in the position adjustment screen. Then, the images that are not displayed in the position adjustment screen are the image 703a and the image 704a. Both the image 703a and the image 704a are located on the lower side with respect to the operation target image 702a and are subjected to the interlocking operation for the operation on the operation target image. Also, in FIG. 7B, when it is assumed that the image 703b is the operation target image, the images that are not displayed in the position adjustment screen are the image 701b and the image 704b, but the image located on the lower side with respect to the operation target image 703b is the image 704b. Therefore, the image 704b becomes the interlocking operation target. Also, in FIG. 7C, when it is assumed that an image 703c is the operation target image, the image that is not displayed in the position adjustment screen is the image 704c. As the image 704c is located on the lower side with respect to the operation target image 703c, the image 704c becomes the interlocking target.

Also, in the case of FIG. 7E, when it is assumed that the image 702e is the currently operated image, it is possible to determine that the image 702e is at the uppermost position and also at the lowermost position in the upper and lower position check in the position adjustment screen in step S504. Therefore, among the images that are not displayed in the position adjustment screen, the images located on the lower side with respect to the currently operated image 702e are the image 703e and the image 704e, and these images become the interlocking targets.

(Step S509) Step S509 corresponds to the interlocking operation, but the processing in the present step is similar to that of step S506.

The processes according to the present embodiment have been described in the above. Through the above-described processing, the appropriate picked-up image group is interlocked and moved without explicitly specifying the picked-up image group that should be interlocked at the time of the position adjustment by the operator. Therefore, it is possible to alleviate the time and effort of the operator in relation to the position adjustment.

It is noted that according to the above-described embodiment, the case has been described in which a registration of the picked-up images is vertical, but the registration of the picked-up images may be horizontal alternatively or additionally to being vertical. In this case, like the above-described processing, it is determined whether the picked-up image that is the operation target is located at a leftmost position or a rightmost position. In a case where the picked-up image that is the operation target is located at the rightmost position, the picked-up image located on the right side with respect to the picked-up image that is the operation target is interlocked and moved. In a case where the picked-up image that is the operation target is located at the leftmost position, the picked-up image located on the left side with respect to the picked-up image that is the operation target is interlocked and moved.

Also, in step S503, the determination is performed as is the case where the image is not displayed in the position adjustment screen, and the determination processing is performed to determine whether the position is the upper position or the lower position with respect to the operation screen in step S505 or step S508. However, the image overlapped with the operation screen may be interlocked. For example, in FIG. 7A, when it is supposed that the image 702a is the currently operated image, the image 702a is at the lowermost position among the images displayed in the screen. Thus, the image 703a and the image 704a which are on the lower side with respect to the image 702a and overlapped with each other are interlocked. Also, in another example, in FIG. 7B, when it is supposed that the image 702b is the currently operated image, as the image 702b is at the uppermost position among the displayed images, the image 701b that is the image overlapped with the image 702b and is located above the image 702b is interlocked and operated.

It is noted that with regard to a workflow at the time of occurrence of an image pickup failure or the like, in a case where Suspend (image pickup suspension) is set, it is presumed that a patient is moved. As it is difficult to reproduce a posture before the suspension, Resume (continued image pickup) may be disabled. (Density step correction between respective images in combining processing in long image pickup)

In a registration window, partial images after the image processing applied on each of the images may be set as blending (translucent) in an overlapped position.
In this case, a seam (density step) is conspicuous. However, it is actually easier for the operator to recognize the seam and manually perform a small position adjustment. After the registration, unprocessed images are first affixed, and a uniform image processing is applied on the combined image, so that the density step between the respective images is not conspicuous.

### (Manual combining from arbitrary selected image)

To enable to execute the registration and the image combining, two restrictions may be prepared. That is, an error is issued in the registration for a case that does not satisfy the following restrictions, and the process does not proceed to the image combining. The first is that an overlapping part with an adjacent image exists in the respective partial images. The second is that no part exists where three or more images are overlapped.

### (Restriction items with respect to image pickup condition and the like)

To make the density step in the combined image less conspicuous, image pickup conditions for the respective partial images are preferably as close to one another as possible. At least, an identical tube voltage is set.

In step S201 according to the present embodiment, the selection of the image pickup protocol is carried out, but for the selection of the image pickup protocol, in addition to the method of manually selecting the protocol, it is possible to carry out collection and selection by using a function of a modality work list defined by the DICOM standard. The obtained modality work list is referred to as image pickup order and is composed of a combination of patient information and the image pickup protocol. This work list may be selected from a list through a manual operation as a list is displayed on the screen or may also be retrieved from a list by using an external interface such as a barcode or a magnetic card. As a retrieval key to be retrieved, a patient ID, a receipt number, or the like is used in many cases. By using a result of reading the barcode or the magnetic card as the retrieval key, the image pickup order is retrieved from the list. At this time, if the image pickup order is not found out, furthermore, by using the result of reading the barcode or the magnetic card as the retrieval key, the work list may be obtained from a server.

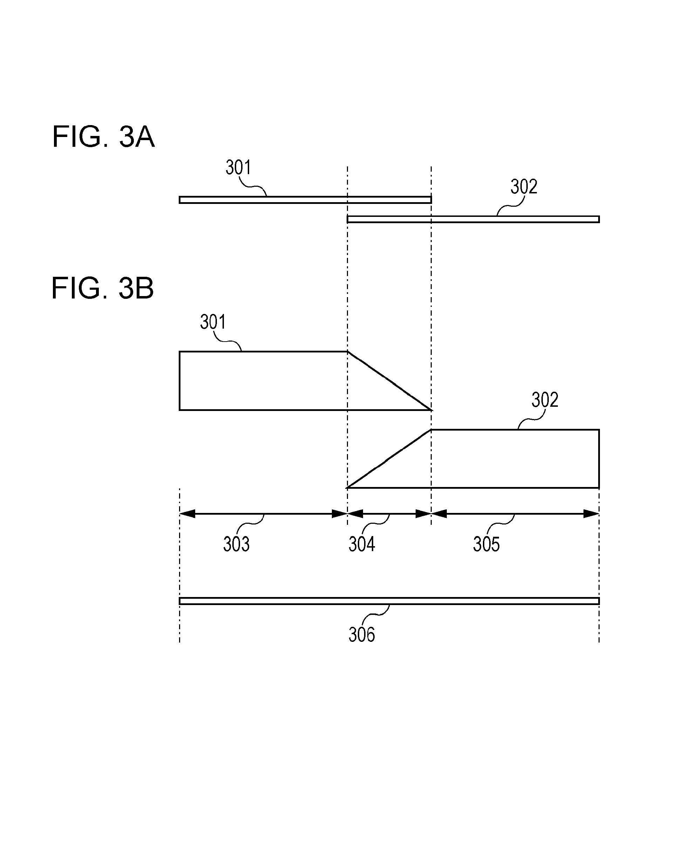

An example of combining stitches will be described by using FIGS. 3A and 3B. In FIGS. 3A and 3B, an image 301 and an image 302 are representations when images are viewed from the side. Herein, the image 301 and the image 302 are set as images to be combined with each other, and an overlapping amount corresponds to an area 304 of FIG. 3B. FIG. 3B is a schematic diagram representing histograms of pixel values of the image 301 and the image 302. If a combining result is set to be a combined image 306, an area 303 is image data of the image 301, and an area 305 is image data of the image 302. In the area 304, as illustrated in the drawing, a pixel data value is calculated so that the image data histogram starts from 100% to 0% while following from a starting position of the image overlap to an end of the image. Also, similarly, in the image 302, a pixel data value is calculated so that the image data histogram starts from 0% to 100% while following from the edge of the image to the starting position of the image overlap. As a result, the image 301 and the image 302 are combined with each other to form the combined image 306.

With regard to the image pickup of the stitch images, prior to a still image pickup, a transillumination inspection may be carried out in some cases. At this time, transillumination dye used prior to the still image pickup is accumulated in the relevant still image and received from an X-ray system to an image pickup PC via a serial communication command. Alternatively, in a case where a plurality of inspections are carried out at the same time, at an ending stage of the inspections, a total dose used in the respective inspections is received from the X-ray system to the image pickup PC via the serial communication command.

### Other Embodiments

Aspects of the present invention can also be realized by a computer of a system or apparatus (or devices such as a CPU or MPU) that reads out and executes a program recorded on a memory device to perform the functions of the above-described embodiment(s), and by a method, the steps of which are performed by a computer of a system or apparatus by, for example, reading out and executing a program recorded on a memory device to perform the functions of the above-described embodiment(s). For this purpose, the program is provided to the computer for example via a network or from a recording medium of various types serving as the memory device (e.g., computer-readable medium).

While the present invention has been described with reference to exemplary embodiments, it is to be understood that the invention is not limited to the disclosed exemplary embodiments, but is encompassed by the scope of the following claims.

## Claims

1. An image processing apparatus for performing a position adjustment on an image displayed on a display screen (106), the image processing apparatus comprising:
obtaining means (101, 102, 103, 104) configured to obtain a plurality of images for which mutual positional relationships are set;
display control means (105) configured to display at least one image among the plurality of images on a display screen;
first position adjustment means (105) configured to adjust a position of the image displayed on the display screen; and
second position adjustment means (105) configured to adjust a position of the image that is not displayed on the display screen among the plurality of images in accordance with the adjustment of the position by the first position adjustment means.

2. The image processing apparatus according to Claim 1, further comprising:
determination means (105) configured to determine whether or not the image of which the position is adjusted by the first position adjustment means is located at an uppermost position or a lowermost position among obtained images displayed in the display screen,
wherein, in a case where the determination means determines that the image is located at the uppermost position, in accordance with the adjustment of the position by the first position adjustment means, the second position adjustment means is configured to adjust a position of an image located above the image of which the position is adjusted by the first position adjustment means among the plurality of images, and
wherein, in a case where the determination means determines that the image is located at the lowermost position, in accordance with the adjustment of the position by the first position adjustment means, the second position adjustment means is configured to adjust a position of an image located below the image of which the position is adjusted by the first position adjustment means among the plurality of images.

3. The image processing apparatus according to Claim 2,
wherein, in a case where the determination means determines that the image of which the position is adjusted by the first position adjustment means is not located at the uppermost position or the lowermost position in the display screen, the second position adjustment means is configured to restrict an adjustment of positions of the plurality of images not displayed in the display screen.

4. The image processing apparatus according to any one of Claims 1 to 3, further comprising:
determination means (105) configured to determine whether or not the image of which the position is adjusted by the first position adjustment means is located at a leftmost position or a rightmost position among picked-up images displayed in the display screen,
wherein, in a case where the determination means determines that the image is located at the leftmost position, in accordance with the adjustment of the position by the first position adjustment means, the second position adjustment means is configured to adjust a position of an image located on a left side with respect to the image of which the position is adjusted by the first position adjustment means among the plurality of images, and
wherein, in a case where the determination means determines that the image is located at the rightmost position, in accordance with the adjustment of the position by the first position adjustment means, the second position adjustment means is configured to adjust a position of an image located to the right of the image of which the position is adjusted by the first position adjustment means among the plurality of images.

5. The image processing apparatus according to Claim 4,
wherein, in a case where the determination means determines that the image of which the position is adjusted by the first position adjustment means is not located at the leftmost position or the rightmost position in the display screen, the second position adjustment means is configured to restrict an adjustment of positions of the plurality of images not displayed on the display screen.

6. The image processing apparatus according to any one of Claims 1 to 5,
wherein, in a case where the adjustment of the position is performed by an amount larger than a predetermined amount by the first position adjustment means, in accordance with the adjustment of the position by the first position adjustment means, the second position adjustment means is configured to adjust positions of the plurality of images.

7. The image processing apparatus according to any one of Claims 1 to 6, further comprising:
combining processing means (105) configured to combine the plurality of images,
wherein the second position adjustment means is configured to restrict an adjustment of the positions after the combining processing means has performed the combining.

8. The image processing apparatus according to any preceding claim, wherein:
the obtaining means (101, 102, 103, 104) is configured to obtain a first image, a second image for which a positional relationship with the first image is set, and a third image;
the display control means (105) is configured to display the first image and the third image on the display screen (106);
the first position adjustment means (105) is configured to adjust a position of the first image with respect to the third image on the display screen; and
the second position adjustment means (105) is configured to adjust a position of the second image so as to maintain the positional relationship with the first image in accordance with the adjustment of the position of the first image by the first position adjustment means.

9. An image processing method of performing a position adjustment on an image displayed on a display screen, the image processing method comprising:
obtaining a plurality of images for which mutual positional relationships are set;
displaying at least one image among the plurality of images on a display screen;
adjusting a position of the image displayed on the display screen; and
adjusting a position of the image that is not displayed on the display screen among the plurality of images in accordance with the adjustment of the position.

10. The image processing method according to Claim 9, further comprising:
obtaining a first image (702b), a second image (701b) of which a positional relation with the first image is set, and a third image (703b);
displaying the first image and the third image on the display screen;
adjusting a position of the first image with respect to the third image on the display screen; and
adjusting a position of the second image so as to maintain the positional relationship with the first image in accordance with the adjustment of the position of the first image.

11. A computer program for causing a computer to function as an image processing apparatus according to any one of Claims 1 to 8.

12. A computer program which, when run on a computer, causes the computer to perform the method of Claim 9 or 10.

13. A storage medium having stored thereon a computer program according to Claim 11 or 12.
